# EUROPÄISCHE PATENTANMELDUNG

(11) **EP 2 138 824 A2**
(43) Veröffentlichungstag der Anmeldung: **30.12.2009**
(21) Anmeldenummer: 09007853.6
(22) Anmeldetag: 16.06.2009
(51) Int. Cl.: G01N 15/06

(54) **Verfahren zum Bestimmen des Eindringens von Prüfpartikeln in einen Messbereich**

(30) Priorität: 24.06.2008 DE 102008029700
(71) Anmelder: Palas GmbH Partikel-und Lasermesstechnik, 76229 Karlsruhe (DE)
(72) Erfinder: Abele, Martin, D-76139 Karlsruhe (DE); Bankodad, Ahmeed, D-42105 Wuppertal (DE); Haep, Stefan, D-46539 Dinslaken (DE); Mölter, Leander, D-76744 Wörth (DE); Munzinger, Friedrich, D-75053 Gondelsheim (DE); Opiolka, Siegfried, D-47239 Duisburg (DE)
(74) Vertreter: Lempert, Jost

(57) **Zusammenfassung**

Zum Bestimmen des Eindringens von Prüfpartikeln in einen Messbereich wird ein Verfahren vorgeschlagen, nach dem fluoreszierende Prüfpartikel außerhalb des Messbereichs ausgegeben werden, die Prüfpartikel innerhalb einer Messeinrichtung mit Fluoreszenz anregender Strahlung bestrahlt werden und die von den Prüfpartikeln emittierte Fluoreszenzstrahlung detektiert wird und die Fluoreszenz emittierten Partikel gezählt werden. Ein System zum Bestimmen des Eindringens von Prüfpartikeln in einen Messbereich ist entsprechend ausgebildet.

## Beschreibung

Die Erfindung betrifft ein Verfahren zum Bestimmen des Eindringens von Prüfpartikeln in einen Messraumbereich.

Zur Messung der Anzahlkonzentration und Größenverteilung von Aerosolen werden optische Partikelzähler eingesetzt. Diese bieten die Vorteile von kurzen Messzeiten, direkter Ergebnisdarstellung und einfacher Handhabung.

Allerdings lassen diese Anordnungen keine Unterscheidung zwischen unterschiedlichen Aerosolen zu. Dieses nicht selektive Zählen und Klassieren ist dann von Nachteil, wenn ein bestimmtes Prüfaerosol in Umgebungsluft eindeutig detektiert und zum Beispiel seine Anzahlkonzentration bestimmt werden muss. Die zeitgleichen ubiquitär vorhandenen weiteren natürlichen und nicht-natürlichen Aerosole in der Umgebungsluft verfälschen die Messung oder machen diese unmöglich. Sie sind daher beispielsweise nicht zur Prüfung des Rückhaltevermögens an der Arbeitsöffnung einer Sicherheitswerkbank nach DIN EN 12469 oder einer Zytostatika-Werkbank nach DIN 12980 (im Folgenden zusammengefasst als Sicherheitswerkbank) in mikrobiologischen oder biotechnischen Laboratorien sowie medizinischen Einrichtungen einsetzbar, da sich dort zusätzlich zu den während der Prüfung freigesetzten Prüfpartikeln auch weitere natürlicherweise vorhandene Partikel, wie zum Beispiel Staub, Abrieb aus Kleidung und Geräten, Hautschuppen usw. innerhalb des Aufstellungsraums der Sicherheitswerkbank befinden. Aus diesem Grunde werden zurzeit für die beschriebene Prüfung von Sicherheitswerkbänken zeitaufwändige und mit prinzipiellen Nachteilen behaftete mikrobiologische und chemische Verfahren zum selektiven Nachweis von Prüfpartikeln eingesetzt. Zur Schutzgradbestimmung von Operationssälen sind zwar Messungen der Eintragsbelastung in dem Operationsbereich mittels Tröpfchenaerosolen vorgeschrieben, praktikable Verfahren zur Unterscheidung eines Prüfaerosols von ubiquitären Teilchen stehen aber nicht zur Verfügung.

Der Erfindung liegt daher die Aufgabe zugrunde, unter Vermeidung der vorgenannten Nachteile ein Verfahren zum Bestimmen des Eindringens von Prüfpartikeln in solche Messraumbereiche vorzuschlagen.

Erfindungsgemäß wird die genannte Aufgabe bei einem gattungsgemäßen Verfahren dadurch gelöst,
- dass fluoreszierende Prüfpartikel außerhalb des Messraumbereichs ausgegeben werden,
- dass die Prüfpartikel innerhalb einer Messeinrichtung mit Fluoreszenz anregender elektromagnetischer Strahlung bestrahlt werden,
- dass die von den Prüfpartikeln emittierte Fluoreszenzstrahlung detektiert wird und
- dass die Fluoreszenz emittierenden Prüfpartikel gezählt werden.

Abgesehen davon, dass die Messeinrichtung in einem hinreichenden Abstand vom Prüfpartikelerzeuger (Aerosolerzeuger) aufgestellt wird, ist weiterhin vorgesehen, dass die Messeinrichtung auch in einem bestimmten Abstand zu einer Messraumgrenze des Messraumbereichs, wie beispielsweise der Öffnung einer Sicherheitswerkbank aufgestellt wird, da der Arbeitsbereich sich ebenfalls in einem entsprechenden Abstand zur Messraumgrenze befindet und in diesem die Wirksamkeit des Schutzes nachzuweisen ist.

In bevorzugter Weiterbildung kann dabei vorgesehen sein, dass Umgebungsluft mit so einem geringen Sog in die Messeinrichtung eingesogen wird, dass sich an der Messraumgrenze keine durch den Sog bewirkte - messbare - Strömung ergibt, wobei insbesondere Umgebungsluft sich mit einem Volumenstrom von 5 bis 30 Liter pro Minute durch die Messeinrichtung bewegt. Ein Ventilator zum Ansaugen der Umgebungsluft in die Messeinrichtung ist entsprechend ausgelegt.

Alternative Ausgestaltungen des Verfahrens sehen vor, dass die Messeinrichtung innerhalb einer Sicherheitswerkbank als Messraumbereich mit endlichem Abstand zu einer Arbeitsöffnung derselben angeordnet wird, dass die Messanordnung außerhalb einer Sicherheitswerkbank zum Arbeiten mit kontaminierten Partikeln mit einem endlichen Abstand zu einer Arbeitsöffnung der Sicherheitswerkbank angeordnet wird oder dass die Messeinrichtung in einem Operationsraum auf einem Operationstisch angeordnet wird.

In bevorzugter Weise werden Partikel eingesetzt, die eine Fluoreszenzanregungswellenlänge in einem Wellenlängenteilbereich eines Bereichs von 400 bis 800 nm, vorzugsweise 400 bis 530 nm aufweisen und eine Fluoreszenz-Emissionswelle an einer Linie in einem Wellenlängenbereich zwischen 440 und 800 nm, vorzugsweise 480 und 550 nm haben. Demgemäß ist weiterhin erfindungsgemäß vorgesehen, dass fluoreszierende Prüfpartikel, die in einem Wellenlängenteilbereich eines Wellenlängenbereichs von 400 bis 780 nm, vorzugsweise 400 bis 530 nm zur Fluoreszenz anregbar sind, ausgegeben und mit Licht im genannten Wellenlängenteilbereich angeregt werden und/oder, dass die Strahlung im Emissionsbereich der Partikel innerhalb eines Wellenlängenbereichs von 440 bis 800 nm, vorzugsweise 480 bis 550 nm detektiert wird. Während die Anregungsstrahlung durch eine schmalbandige Lichtquelle, wie einen LED-Laser oder dergleichen erzeugt wird, kann auch eine Lichtquelle weißen Lichts mit einem geeigneten Filter im Anregungsstrahlgang eingesetzt werden.

Damit erfolgt die Fluoreszenzanregung durch Lichtquellen, insbesondere Laser, wie Laser und LED im noch optischen bzw. nahen UV-Bereich, bei denen in das Messvolumen innerhalb der Messeinrichtung noch eine hinreichende Anregungssintensität einspeisbar ist.

In weiterer bevorzugter Ausgestaltung ist vorgesehen, dass Fluoreszenzstrahlung in der gleichen Ebene senkrecht zurStrömungsrichtung der Prüfpartikel detektiert wird, in der die Prüfpartikel im Messvolumen innerhalb der Messeinrichtung durch die Messstrahlung angeregt werden. Es hat sich gezeigt, dass im Hinblick auf die Fluggeschwindigkeit der Prüfpartikel die Fluoreszenzanregung nahezu instantan erfolgt, so dass eine Messung innerhalb der gleichen Querschnittsebene senkrecht zur Flugrichtung vorgenommen werden kann und nicht mit endlichem Abstand zum Anregungsort in Strömungsrichtung vorgenommen werden muss. Hierdurch wird eine Vereinfachung des gesamten Aufbaus erreicht. Hierzu trägt ebenfalls bei, dass die emittierte Fluoreszenzstrahlung unter einem Winkel ungleich Null und ungleich 180°, vorzugsweise im Bereich zwischen 30 und 120° zur Einstrahlrichtung der Anregungsstrahlung detektiert wird, wobei insbesondere die emittierte Fluoreszenzstrahlung unter einem Winkel von 90° zur Einstrahlrichtung der Anregungsstrahlung detektiert wird. Um jeglichen Anteil von Anregungsstrahlung und Umgebungslicht im Detektor auszuschließen, sieht eine bevorzugte Ausgestaltung vor, dass die emittierte Fluoreszenzstrahlung zur Detektion durch einen Filter hindurchtritt, der diese Strahlung hindurchtreten lässt, andere Wellenlängen, insbesondere das Licht der Anregungsstrahlung aber blockiert. Der Aufbau der Vorrichtung kann weiterhin dadurch vereinfacht werden, dass Licht in das Messvolumen der Messeinrichtung lediglich durch transparente, nicht aber spiegelnde Optiken eingebracht wird. Somit kann ein konstruktiv einfaches dreidimensionales Messvolumen verwendet werden, das eine einfache und schnelle Aufnahme der Prüfaerosole gewährleistet.

Bevorzugte Weiterbildungen sehen vor, dass Prüfpartikel mit Größen von 0,5 bis 10 Mikrometer ausgebracht werden und dass ein Aerosol der Prüfpartikel mit einer Konzentration von 10² bis 10⁶ Partikel pro Kubikzentimeter erzeugt wird und/oder dass mittels der Messeinrichtung Partikelkonzentrationen von einem Partikel pro Kubikzentimeter bis 10⁵ Partikel pro Kubikzentimeter gemessen werden.

Weiterhin ist bevorzugt vorgesehen, dass die Prüfpartikel mit einer Dosierkonstanz von einer Stunde und mehr erzeugt werden.
Weitere Vorteile und Merkmale der Erfindung ergeben sich aus den Ansprüchen und aus der nachfolgenden Beschreibung, in der Ausführungsbeispiele der Erfindung unter Bezugnahme auf die Zeichnung im Einzelnen erläutert sind. Dabei zeigt:

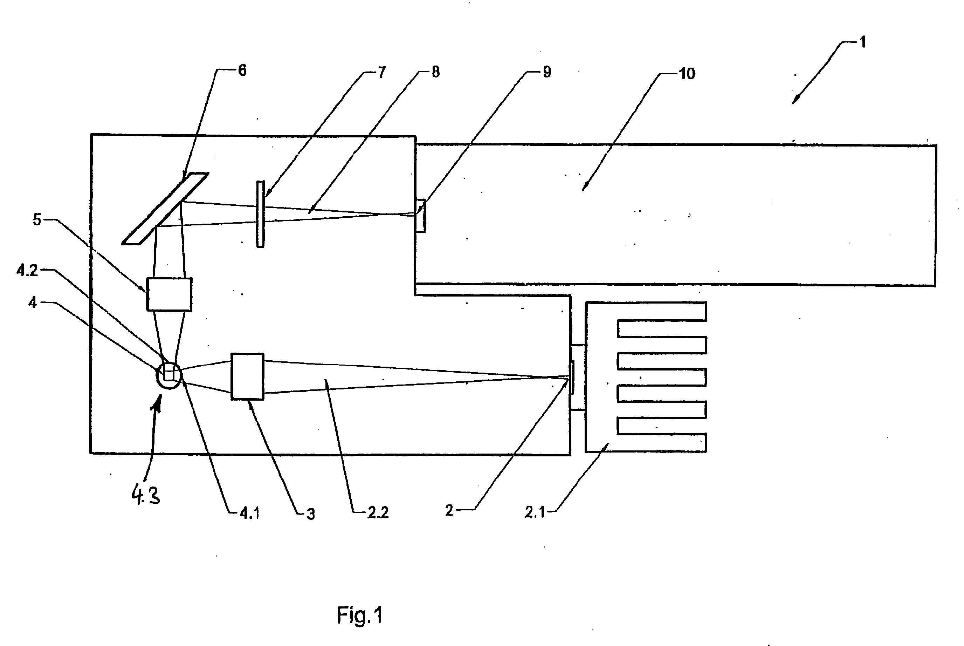

- Fig. 1: eine erfindungsgemäße Messeinrichtung in schematischer Darstellung;
- Fig. 2: eine Sicherheitswerkbank mit Anordnung eines Detektors gemäß Fig.1 innerhalb der Werkbank;
- Fig. 3: eine Sicherheitswerkbank mit Anordnung eines Detektors gemäß Fig. 1 außerhalb der Werkbank;
- Fig. 4: eine schematische Darstellung eines Operationsbereichs in Frontansicht; und
- Fig. 5: eine schematische Darstellung des Operationsbereichs der Fig. 4 in Draufsicht.

Die erfindungsgemäße Messeinrichtung 1 weist eine Lichtquelle 2 auf, die vorzugsweise als Laser-LED im blauen Bereich bei etwa 400 Nanometer Lichtwellenlänge (genau 405 nm) ausgebildet ist, obwohl grundsätzlich auch andersartige Laser oder Lichtquellen mit "weißem Licht" in Frage kommen; in letzterem Falle wird ein Filter vorzusehen sein, um die geeignete Anregungswellenlänge durchzulassen, alle anderen Wellenlängen aber auszufiltern.

Der Lichtquelle 2 ist eine Kühlanordnung 2.1 zugeordnet. Im Lichtstrahl 2.2 der Lichtquelle 2 findet sich eine Sammellinse in Form eines Kollimators 3, so dass durch das Licht ein Messvolumen 4 der Messeinrichtung 1 in einem Rohr 4.3 beleuchtet wird, in dem die fluoreszierenden Teilchen senkrecht zur Blattfläche und damit senkrecht zum Strahl 2.2 strömen. Neben dem Eintrittsfenster 4.1 für den Lichtstrahl weist das Messvolumen 4 ein Austrittsfenster 4.2 auf, aus dem Fluoreszenzstrahlung der Messpartikel austritt, durch eine Sammellinse 5 auf einen Spiegel 6 gerichtet, von diesem bei der gegebenen Anordnung umgelenkt und durch einen Filter 7, der hier ein Grünfilter ist und lediglich die Fluoreszenzwellenlänge der Fluoreszenzpartikel hindurch lässt, anderes Licht aber absperrt, gelenkt wird. Der Fluoreszenzstrahl 8 tritt auf einen Detektor 9 innerhalb einer Zählelektronik 10, mittels derer die Fluoreszenzteilchen gezählt werden. Das dreidimensionale Messvolumen 4 ist definiert durch den Raumbereich im von den Teilchen durchströmten Rohr 4.3, aus dem Fluoreszenzlicht der bestrahlten Partikel durch das Fenster 4.2 austritt und als Maßstrahlung detektiert wird.

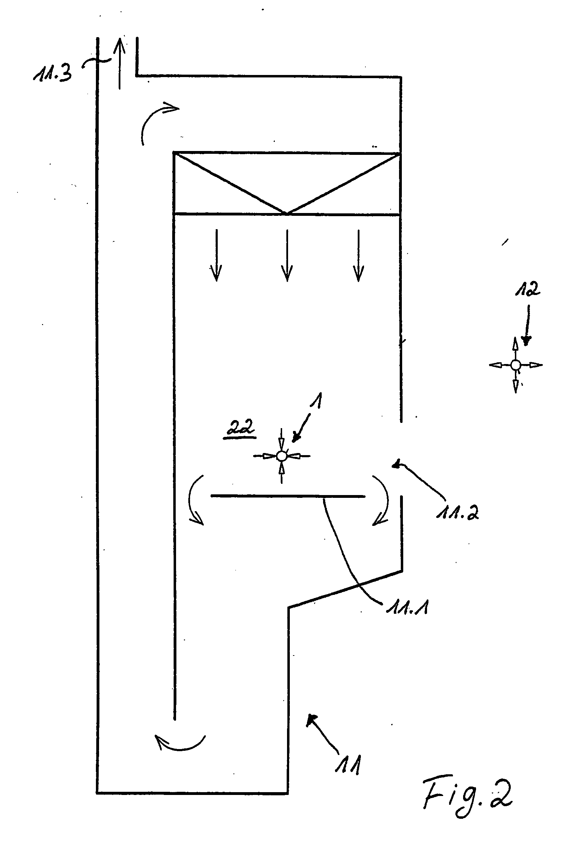

Die Fig. 2 zeigt eine Sicherheitswerkbank 11 mit einer Arbeitsfläche 11.1 und einer Arbeitsöffnung 11.2, durch welche die Arbeitsfläche 11.1 zugänglich ist sowie einem Luftführungssystem mit einem Ventilator (nicht dargestellt), das die Luft teilweise als Umluft innerhalb der Sicherheitswerkbank 11 umwälzt, teilweise über einen Auslass 11.3, dem gegebenenfalls ein Filter vorgeschaltet ist (nicht dargestellt) in die Umgebung abgibt.

Bei der Darstellung der Fig. 2 findet sich eine erfindungsgemäße Messeinrichtung 1 innerhalb der Sicherheitswerkbank, während in einem Messraumbereich 22 außerhalb der Sicherheitswerkbank ein Aerosol- oder Partikelgenerator 12 vorgesehen ist, der ein Aerosol fluoreszierender Teilchen in an sich bekannter Weise erzeugt. Durch die in der Fig. 2 dargestellte Anordnung kann damit festgestellt werden, in welchem Umfange Prüf- oder Fluoreszenzpartikel aus der Umgebung in die Sicherheitswerkbank und damit in den durch den Arbeitsbereich auf der Arbeitsplatte 11.1 definierten Messraumbereich gelangen können, die für vom Außenraum in die Sicherheitswerkbank eintretende Kontaminationen oder Verunreinigungen beim Arbeiten in der Sicherheitswerkbank repräsentativ sind.

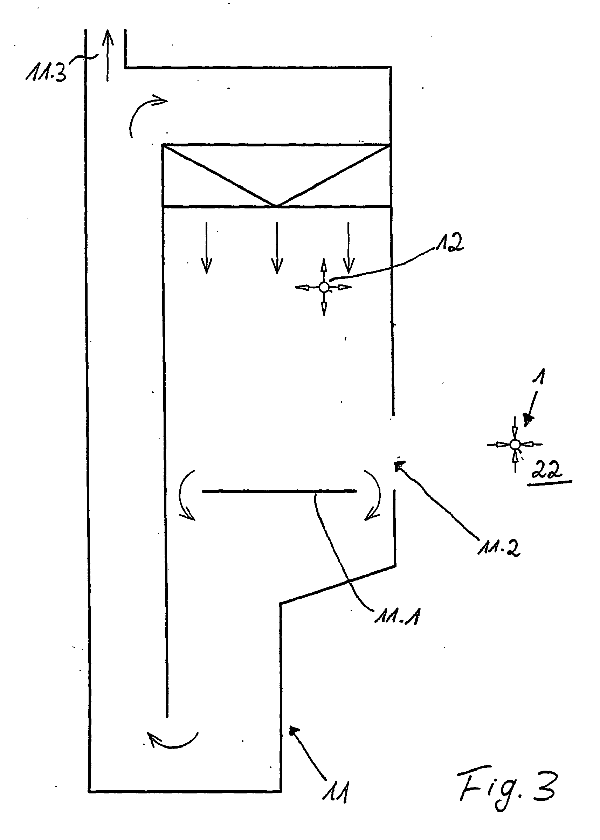

Die Fig. 3 zeigt die gleiche Sicherheitswerkbank 11, wobei bei der hier dargestellten Anordnung davon ausgegangen wird, dass innerhalb der Sicherheitswerkbank mit kontaminierenden Stoffen gearbeitet wird. Demgemäß findet sich eine Messeinrichtung 1 mit einem Partikelzähler in einem Messraumbereich 22 außerhalb der Sicherheitswerkbank 11 mit hinreichendem Abstand vor der Arbeitsöffnung 11.2, während der Partikelgenerator 12 innerhalb der Sicherheitswerkbank 11 angeordnet ist. Demgemäß kann durch diese erfindungsgemäße Anordnung festgestellt werden, ob und in welchem Umfange Prüf- bzw. Fluoreszenzteilchen, die kontaminierende Teilchen beim Arbeitsprozess mit der Sicherheitswerkbank 11 repräsentieren, aus dieser heraus und in die Umgebung eintreten und dort gegebenenfalls zu Kontaminationen führen können.

Die erfindungsgemäße Messeinrichtung 1 kann einen Ventilator aufweisen, mit dem Luft und in dieser befindlicher Teilchen, einschließlich Prüf- oder Aerosolteilchen durch das Messvolumen 4 hindurchgesaugt werden, wobei der Sog allerdings so gering ist, dass an der Arbeitsöffnung 11.2 der Sicherheitswerkbank jeweils kein durch diesen Ventilator bewirkter messbarer Sog entsteht.

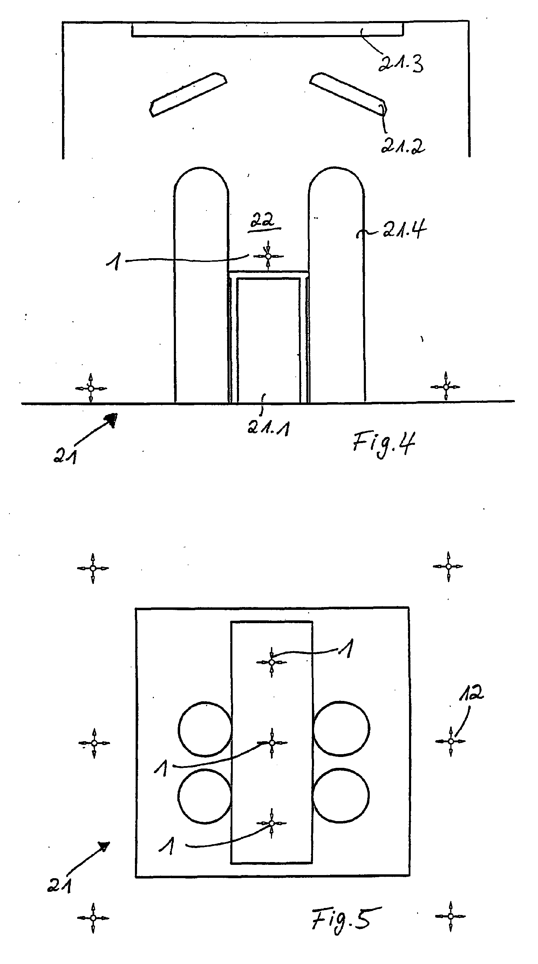

Die Fig. 4 und 5 zeigen eine weitere erfindungsgemäße Anordnung in Form eines Operationsraumes 21. Dieser weist einen Operationstisch 21.1, Operationsleuchten 21.2 sowie eine Zuluftdecke 21.3 auf, durch die - reine - Zuluft in den Operationssaal eingeleitet werden kann.

Weiterhin werden zur Prüfung Dummies 21.4 vorgesehen, die Personen bei einer Operation - Ärzte, Operationshilfskräfte - hinsichtlich Temperatur- und Feuchtigkeitseinbringung sowie Einfluss auf das Strömungsverhalten der Luft im Operationsraum 21 repräsentieren.

In einem solchen Operationsraum ist zu überprüfen, dass Verunreinigungspartikel nicht in den Bereich des auf dem Operationstisch 21.1 liegenden Patienten gelangen, wobei dieser Bereich auch hier wieder einen Messraumbereich 22 bildet. Demgemäß werden an geeigneten Stellen im Raum Messeinrichtungen 1 angeordnet.

So kann erfindungsgemäß festgestellt werden, in welchem Umfange durch die Partikelgeneratoren 12 erzeugte Prüf- bzw. Fluoreszenzpartikel, die im Operationsraum befindliche Kontaminationen, sei es eingedrungen durch Zugangstüren, sei es abgesondert von im Raum befindlichen Personen in dem Operationsbereich, in dem sich die Messeinrichtungen 1 befinden, gelangen können.

### Bezugszeichenliste

- 1: Messeinrichtung
- 2: Lichtquelle
- 2.1: Kühlanordnung
- 2.2: Lichtstrahl
- 3: Kollimator
- 3.5: Optiken
- 4: Messvolumen
- 4.1: Eintrittsfenster
- 4.2: Austrittsfenster
- 4.3: Rohr
- 5: Sammellinse
- 6: Spiegel
- 7: Filter
- 8: Fluoreszenzstrahl
- 9: Detektor
- 10: Zählelektronik
- 11: Sicherheitswerkbank
- 11.1: Arbeitsfläche
- 11.2: Arbeitsöffnung
- 11.3: Auslass

- 12: Partikelgenerator
- 21: Operationsraum
- 21.1: Operationstisch
- 21.2: Operationsleuchten
- 21.3: Zuluftdecke
- 21.4: Dummies
- 22: Messbereich

## Patentansprüche

1. Verfahren zum Bestimmen des Eindringens von Prüfpartikeln in einen Messraumbereich, **dadurch gekennzeichnet,**
- **dass** fluoreszierende Prüfpartikel außerhalb des Messraumbereichs ausgegeben werden,
- **dass** die Prüfpartikel innerhalb einer Messeinrichtung mit Fluoreszenz anregender elektromagnetischer Strahlung bestrahlt werden,
- **dass** die von den Prüfpartikeln emittierte Fluoreszenzstrahlung detektiert wird und
- die Fluoreszenz emittierenden Prüfpartikel gezählt werden.

2. Verfahren nach Anspruch 1, **dadurch gekennzeichnet, dass** die Messeinrichtung in einem bestimmten endlichen Abstand zur Grenze des Messraums angeordnet wird.

3. Verfahren nach Anspruch 1 oder 2, **dadurch gekennzeichnet, dass** Umgebungsluft mit so einem geringen Sog in die Messeinrichtung eingesogen wird, dass sich an der Messraumgrenze keine durch den Sog bewirkte - messbare - Strömung ergibt.

4. Verfahren nach Anspruch 3, **dadurch gekennzeichnet, dass** Umgebungsluft sich mit einem Volumenstrom von 25 bis 30 Liter pro Minute durch die Messeinrichtung bewegt.

5. Verfahren nach einem der vorangehenden Ansprüche, **dadurch gekennzeichnet, dass** die Messeinrichtung innerhalb einer Sicherheitswerkbank mit endlichem Abstand zu einer Arbeitsöffnung derselben angeordnet wird.

6. Verfahren nach einem der Ansprüche 1 bis 4, **dadurch gekennzeichnet, dass** die Messanordnung außerhalb einer Sicherheitswerkbank zum Arbeiten mit kontaminierten Partikeln mit einem endlichen Abstand zu einer Arbeitsöffnung der Sicherheitswerkbank angeordnet wird.

7. Verfahren nach einem der Ansprüche 1 bis 4, **dadurch gekennzeichnet, dass** die Messeinrichtung in einem Operationsraum auf einer Operationsliege angeordnet wird.

8. Verfahren nach einem der vorangehenden Ansprüche, **dadurch gekennzeichnet, dass** fluoreszierende Prüfpartikel, die in einem Wellenlängenteilbereich eines Wellenlängenbereichs von 400 bis 780 nm, vorzugsweise 400 bis 530 nm zur Fluoreszenz anregbar sind, ausgegeben und mit Licht im genannten Wellenlängenteilbereich angeregt werden.

9. Verfahren nach einem der vorangehenden Ansprüche, **dadurch gekennzeichnet, dass** die Strahlung im Emissionsbereich der Partikel innerhalb eines Wellenlängenbereichs von 440 bis 800 nm, vorzugsweise 480 bis 550 nm detektiert wird.

10. Verfahren nach einem der vorangehenden Ansprüche, **dadurch gekennzeichnet, dass** Fluoreszenzstrahlung in der gleichen Ebene senkrecht zur Strömungsrichtung der Prüfpartikel detektiert wird, in der die Prüfpartikel durch die Messstrahlung angeregt werden.

11. Verfahren nach einem der vorangehenden Ansprüche, **dadurch gekennzeichnet, dass** die emittierte Fluoreszenzstrahlung unter einem Winkel ungleich Null und ungleich 180°, vorzugsweise im Bereich zwischen 30 und 120° zur Einstrahlrichtung der Anregungsstrahlung gesammelt wird.

12. Verfahren nach Anspruch 11, **dadurch gekennzeichnet, dass** die emittierte Fluoreszenzstrahlung unter einem Winkel von 90° zur Einstrahlrichtung der Anregungsstrahlung detektiert wird.

13. Verfahren nach einem der vorangehenden Ansprüche, **dadurch gekennzeichnet, dass** die emittierte Fluoreszenzstrahlung zur Detektion durch einen Filter hindurchtritt, der diese Strahlung hindurchtreten lässt, andere Wellenlängen, insbesondere das Licht der Anregungsstrahlung aber blockiert.

14. Verfahren nach einem der vorangehenden Ansprüche, **dadurch gekennzeichnet, dass** Licht in das Messvolumen der Messeinrichtung lediglich durch transparente, nicht aber spiegelnde Optiken eingebracht wird.

15. Verfahren nach einem der vorangehenden Ansprüche, **dadurch gekennzeichnet, dass** Prüfpartikel mit Größen von 0,5 bis 10 Mikrometer ausgebracht werden.

16. Verfahren nach einem der vorangehenden Ansprüche, **dadurch gekennzeichnet, dass** ein Aerosol der Prüfpartikel mit einer Konzentration von 10² bis 10⁶ Partikel pro Kubikzentimeter erzeugt wird.

17. Verfahren nach einem der vorangehenden Ansprüche, **dadurch gekennzeichnet, dass** mittels der Messeinrichtung Partikelkonzentrationen von weniger als einem Partikel pro Kubikzentimeter bis 10⁵ Partikel pro Kubikzentimeter gemessen werden.

18. System zum Bestimmen des Eindringens von Prüfpartikeln in einen Messraumbereich, **gekennzeichnet durch**
- einen Partikelgenerator (12) zum Erzeugen fluoreszierender Prüfpartikel außerhalb des Messraumbereichs (22),
- eine Messeinrichtung (1) mit einer Lichtquelle (2) zum Bestrahlen der Prüfpartikel mit Fluoreszenz anregender elektromagnatischer Strahlung (2.2),
- einen Detektor zum Detektieren von den Prüfpartikeln emittierter Fluoreszenzstrahlung (8)und
- einem Partikelzähler (10) zum Zählen der Fluoreszenz emittierenden Prüfpartikel.

19. System nach Anspruch 18, **dadurch gekennzeichnet, dass** die Messeinrichtung (1) in einem bestimmten endlichen Abstand zur Grenze des Messraumbereichs (22) angeordnet ist.

20. System nach Anspruch 18 oder 19, **gekennzeichnet durch** einen Ventilator zum Ansaugen von Umgebungsluft mit so einem geringen Sog in die Messeinrichtung, dass sich an der Messraumgrenze keine **durch** den Sog bewirkte - messbare - Strömung ergibt.

21. System nach einem der Ansprüche 18 bis 20, **gekennzeichnet durch** eine Sicherheitswerkbank (11) innerhalb der Messeinrichtung (1), die mit endlichem Abstand zu einer Arbeitsöffnung (11.2) der Sicherheitswerkbank angeordnet ist.

22. System nach einem der Ansprüche 18 bis 20, **gekennzeichnet durch** eine Sicherheitswerkbank (11) zum Arbeiten mit kontaminierten Partikeln, außerhalb der die Messeinrichtung (1) mit einem endlichen Abstand zu einer Arbeitsöffnung (11.2) der Sicherheitswerkbank angeordnet ist.

23. System nach einem der Ansprüche 18 bis 20, **gekennzeichnet durch** einen Operationsraum (21), in dem die Messeinrichtung (1) auf einem Operationstisch (21.1) angeordnet ist.

24. System nach einem der Ansprüche 18 bis 23, **dadurch gekennzeichnet, dass** der Partikelerzeuger zur Erzeugung fluoreszierender Prüfpartikel, die in einem Wellenlängenteilbereich eines Wellenlängenbereichs von 400 bis 780 nm, vorzugsweise 400 bis 530 nm zur Fluoreszenz anregbar sind, ausgebildet ist und die Messeinrichtung (1) zur Bestrahlung der Prüfpartikel mit Licht im genannten Wellenlängenteilbereich ausgebildet ist.

25. System nach einem der Ansprüche 18 bis 24, **dadurch gekennzeichnet, dass** die Messeinrichtung (1) zur Detektion der Strahlung im Emissionsbereich der Partikel innerhalb eines Wellenlängenbereichs von 440 bis 800 nm, vorzugsweise 480 bis 550 nm ausgebildet ist und insbesondere vor einem Detektieren einen im Emissionsbereich der Partikel durchlässigen Filter aufweist.

26. Verfahren nach einem der Ansprüche 18 bis 25, **dadurch gekennzeichnet, dass** die Messeinrichtung (1) derart ausgebildet ist, dass die emittierte Fluoreszenzstrahlung unter einem Winkel ungleich Null und ungleich 180°, vorzugsweise im Bereich zwischen 30 und 120° zur Einstrahlrichtung der Anregungsstrahlung (2.2) detektierbar ist.

27. System nach einem der Ansprüche 18 bis 26, **gekennzeichnet durch** lediglich transparente, nicht aber spiegelnde Optiken (3.5).

28. System nach einem der Ansprüche 18 bis 27, **dadurch gekennzeichnet, dass** der Partikelerzeuger (12) zur Erzeugung von Prüfpartikeln mit Größen von 0,5 bis 10 Mikrometer ausgebildet ist.

29. System nach einem der Ansprüche 18 bis 28, **dadurch gekennzeichnet, dass** der Partikelerzeuger (12) zur Erzeugung eines Aerosols der Prüfpartikel mit einer Konzentration von 10² bis 10⁶ Partikel pro Kubikzentimeter ausgebildet ist.

30. System nach einem der Ansprüche 18 bis 29, **dadurch gekennzeichnet, dass** mittels der Messeinrichtung (1) Partikelkonzentrationen von einem Partikel pro Kubikzentimeter bis 10⁵ Partikel pro Kubikzentimeter gemessen werden.
